# EUROPÄISCHE PATENTANMELDUNG

(11) **EP 0 743 518 A1**
(43) Veröffentlichungstag der Anmeldung: **20.11.1996**
(21) Anmeldenummer: 96107978.7
(22) Anmeldetag: 20.05.1996
(51) Int. Cl.: G01N 33/14, G01N 1/10, G01N 1/14

(54) **Verfahren und Vorrichtung zum Bestimmen des Extraktgehaltes von Traubengut bei der Anlieferung an Annahmestationen**

(30) Priorität: 18.05.1995 DE 19518210
(71) Anmelder: Leo Kübler GmbH Thermometer-, Aräometerfabrik, 76133 Karlsruhe (DE); Armbruster, Hermann, 74363 Güglingen (DE)
(72) Erfinder: Alber, Bernd, Dr., D-76227 Karlsruhe (DE); Armbruster, Hermann, D-74363 Güglingen (DE)
(74) Vertreter: Lempert, Jost, Dipl.-Phys. Dr. rer.nat.

(57) **Zusammenfassung**

Zur frühen, statistisch einwandfreien Bestimmung des Extraktgehaltes von zur Weiterverarbeitung angeliefertem Traubengut schlägt die Erfindung ein Verfahren vor, bei dem eine Probe des angelieferten Traubengutes genommen wird, diese Probe gepreßt wird und anhand des ausgepreßten Saftes der Probe der Extraktgehalt bestimmt wird. Als Neuerung ist vorgesehen, daß die Probe mit einem wesentlich höheren Preßdruck ausgepreßt wird als die angelieferten Chargen des Traubengutes zum Erhalt des Traubenmostes gepreßt werden. Die Erfindung sieht zum gleichen Zweck eine Vorrichtung zum Bestimmen des Extraktgehaltes von Traubengut bei der Anlieferung zur Verarbeitung zu Traubenmost mit einer Probennahmeeinrichtung zum Nehmen einer Probe des angelieferten Traubengutes, mit einer Probenpreßeinrichtung zum Pressen der Probe und mit einer Meßeinrichtung zur Bestimmung des Extraktgehaltes der Probe vor, die gekennzeichnet ist durch eine mit einem wesentlich höheren Preßdruck arbeitende Probenpreßeinrichtung, mit dem die angelieferte Charge des Traubengutes zum Erhalt des Traubenmostes gepreßt wird.

## Beschreibung

Die Erfindung betrifft ein Verfahren zum Bestimmen des Extraktgehaltes von Traubengut bei der Anlieferung zur Verarbeitung zu Traubenmost, wobei eine Probe des angelieferten Traubengutes genommen wird, diese gepreßt wird und anhand des ausgepreßten Saftes der Probe der Extraktgehalt bestimmt wird, sowie eine Vorrichtung zum Bestimmen des Extraktgehaltes von Traubengut bei der Anlieferung zur Verarbeitung zu Traubenmost, mit einer Probennahmeeinrichtung zum Nehmen einer Probe des angelieferten Traubengutes, mit einer Probenpreßeinrichtung zum Pressen der Probe und mit einer Meßeinrichtung zur Bestimmung des Extraktgehaltes der Probe.

Anlieferer, meist Angehörige einer Winzergenossenschaft, bringen gelesenes Traubengut in Behältnissen (Butten) zu einer Annahmestation, in der das Traubengut weiterverarbeitet wird. Das Traubengut besteht aus unbeschädigten Trauben samt Kämmen sowie Beeren, Beerenteilen und Saft, sogenanntem Mostvorlauf. Dieser tritt während des Transportes und während des Einschüttens des Traubengutes in einen Annahmetrichter der Annahmestation stets in mehr oder weniger großer Menge aus den Beeren aus. Die ausgetretene Saftmenge hängt von verschiedenen Kriterien ab, wie vom Reifezustand der Beeren, von den Verhältnissen des Transports der Trauben und von der Art der Lese. Bei Traubenvollernten ist der Anteil an Saft, an einzelnen Beeren und an Beerenteilen relativ hoch.

Das angelieferte Traubengut gelangt derzeit in der Regel von dem Aufnahmebehälter über eine Abbeermaschine, wo die Kämme von den Beeren und Beerenteilen getrennt werden, in eine Traubenpresse, in der das Traubengut bis zu mehreren Stunden gepreßt wird. Der austretende Saft setzt sich zusammen aus dem von vorneherein vorhandenen Saft und dem durch das Pressen aus den Beeren austretenden Saft.

Der für die Weiterverarbeitung des Saftes oder Mostes relevante und auch zur Qualifikation des herzustellenden Weines maßgebliche Extraktgehalt, dessen wesentlicher Anteil Zucker ist (der durch Vergärung des Saftes in Alkohol umgesetzt wird) - bewertet in °Oechsle, °MMW u. dgl. -, wird aus dem Mostvorlauf und aus dem Saft oder Most, der durch die Traubenpresse der Kellereieinrichtung erzeugt wurde, als "wahre" Oechslegrade ("wahres" Mostgewicht) bestimmt.

Die Winzer wollen unmittelbar nach der Anlieferung den Extraktgehalt wissen, weil dieser neben dem Gewicht der Anlieferung für den Auszahlungsbetrag entscheidend ist. Dieses Verlangen stößt auf Schwierigkeiten. So ist der durch die Traubenpresse erzeugte Saft oft schon aus einer Mischung von angelieferten Chargen unterschiedlicher Erzeuger (Winzer) hergestellt, weil die Pressenkapazität ausgenützt werden soll. Darüber hinaus dauert das Auspressen des Saftes in der Traubenpresse, wie gesagt, bis zu mehreren Stunden. Andererseits ist die Qualität des vom einzelnen Winzer angelieferten Traubengutes zu bestimmen. Darüber hinaus soll diese Bestimmung auch unmittelbar nach der Anlieferung erfolgen. Eine Bestimmung des Extraktgehaltes aufgrund der Gesamtmenge ist daher nicht, zumindest nicht unmittelbar nach der Anlieferung möglich. Aus diesem Grunde wird unmittelbar nach der Anlieferung, gegebenenfalls nach Abbeeren und leichtem Vorquetschen der Trauben in der Regel unter der Abbeermaschine bzw. am Boden eines Wiegebehälters befindlicher Saft als Probe entnommen und meist refraktometrisch der Annahme-Extraktgehalt bestimmt. Es hat sich bei Prüfungen herausgestellt, daß der so bestimmte Extraktgehalt um mehrere °Oechsle vom wahren Extraktgehalt abweichen kann.

Es wurde auch schon vorgeschlagen, z.B. eine Saftprobe durch Einstechen einer Nadel in die Trauben oder Beeren und Absaugen oder durch Einstoßen eines Rohres an einer Stelle in das Traubengut und undefiniertes Pressen zu erhalten. Auch dies erwies sich in der Praxis als untauglich.

Die Bedeutung des genannten Problems der Abweichung zwischen dem tatsächlichen oder richtigen Extraktgehalt und dem bei der Anlieferung gemessenen wird auch dadurch verdeutlicht, daß ca. 1,2 Mio. Mitglieder Trauben an genossenschaftliche Abnahmestationen liefern.

Für die fehlerhafte Bestimmung des Extraktgehaltes unmittelbar nach der Annahme der Trauben sind im wesentlichen eine fehlerhafte Stichprobennahme und eine fehlerhafte Weiterverarbeitung der Stichprobe verantwortlich. Darüber hinaus können sich Fehler aufgrund fehlerhafter Meßgeräte sowie einer unzulänglichen Reinigung der mit den Proben in Berührung gelangenden Einrichtungen ergeben.

Der Erfindung liegt die Aufgabe zugrunde, unter Vermeidung der vorgenannten Nachteile die Möglichkeit zu schaffen, unmittelbar nach der Annahme einer Charge von Traubengut eine Bestimmung des richtigen Extraktgehaltes der angelieferten Charge zu ermöglichen.

Zur Lösung der genannten Aufgabe sieht die Erfindung bei einem Verfahren der eingangs genannten Art vor, daß die Probe mit einem wesentlich höheren Proben-Preßdruck gegenüber dem Preßdruck ausgepreßt wird, mit dem die gesamte angelieferte Charge des Traubengutes, deren Extraktgehalt vorab bestimmt werden soll, zum Erhalt des Traubenmostes gepreßt wird. Eine gattungsgemäße Vorrichtung sieht zur Lösung der genannten Aufgabe eine mit einem gegenüber dem Preßdruck, mit dem die angelieferte Charge des Traubengutes zum Erhalt des Traubenmostes gepreßt wird, wesentlich höheren Proben-Preßdruck arbeitende Probenpreßeinrichtung vor.

Es wurde festgestellt, daß bei der einzelnen Weinbeere die Extrakt- und Zuckerverteilung über den Querschnitt unterschiedlich ist. Beim Pressen einer Beere wird zuerst Saft aus dem Außenbereich der Beere austreten. Die Probe wird so gepreßt, daß sich in etwa dieselbe Mostausbeute ergibt wie beim Pressen der gesamten angelieferten Charge des Traubengutes, dessen Mostextrakt vorab bestimmt werden soll, d.h. mit einem Ausbeutegrad von 0,70 bis 0,85 cm³/g (Saftmenge pro Traubenmasse), wobei der genaue Ausbeutegrad abhängig ist von Sorte, Reifegrad, Traubenzustand, Kelter u.a. Da das Traubengut "im Keller" über ein bis fünf Stunden mit 1 bis 5 bar gepreßt wird und das Pressen der Probe in einer sehr kurzen Zeit erfolgt, muß der Proben-Preßdruck wesentlich höher als der Keller-Preßdruck sein und sollte mindestens bei mehr als dem Doppelten, vorzugsweise mehr als dem Fünffachen des Keller-Preßdrucks liegen. Wenn daher beim Pressen der Probe nicht dieselben Gesamtverhältnisse herrschen, daß sich derselbe Ausbeutegrad ergibt wie beim Pressen der Gesamtcharge des Traubengutes, so wird der austretende Probensaft einen anderen Extraktgehalt aufweisen als das gesamte Traubengut. Durch die erfindungsgemäße Maßnahme wird der Extraktgehalt richtig bestimmt.

Gemäß einer bevorzugten Ausgestaltung ist vorgesehen, daß ein dünner Preßkuchen erzeugt wird. Hierdurch wird ein sehr schnelles Ablaufen des bei der Probe ausgepreßten Saftes erhalten.

Während grundsätzlich vorgesehen sein kann, daß die Probe diskontinuierlich gepreßt wird, wobei dies durch eine diskontinuierlich arbeitende Probenpreßeinrichtung, wie eine halbkontinuierlich oder taktweise arbeitende Membranpresse, oder durch Quetschventile erfolgen kann, sieht eine äußerst bevorzugte Ausgestaltung vor, daß die Probe kontinuierlich gepreßt wird, wobei dies durch eine kontinuierlich arbeitende Probenpreßeinrichtung, wie insbesondere eine Schneckenpresse geschehen kann.

Damit der aus der Probe ausgepreßte Saft nicht verlorengeht, sieht eine bevorzugte Weiterbildung vor, daß der aus der Probe ausgepreßte Saft nach Bestimmung seines Extraktgehaltes dem Traubengut, zu dessen Vorabbestimmung des Extraktgehaltes er diente, wieder zugeführt wird. Vorrichtungsmäßig ist hierzu eine von der Probenpreßeinrichtung zum Förderweg der Trauben führende Leitung vorgesehen.

Obwohl grundsätzlich, wenn Trauben - bestehend aus Stielgerüst und Traubenbeeren - als Probe entnommen werden, auch das Pressen der Trauben mit ihrem Stielgerüst oder ihren Kämmen erfolgen kann, sieht eine bevorzugte Weiterbildung vor, daß die Beeren der Probe vor dem Auspressen von ihren Kämmen abgebeert und ausgequetscht werden, wobei hierzu der Probenpreßeinrichtung eine Mini-Abbeermaschine und eine Quetschvorrichtung vorgeschaltet sein kann. Das Abbeeren bewirkt eine Minimierung des Gehalts an Gerbstoff und oxydablen Phenolen im durch Pressen erhaltenen Probensaft. Gerbstoff und oxydable Phenole sind vor allem in den grünen Rappen enthalten. Ein zu hoher Anteil dieser Stoffe ist unerwünscht. Durch das Mahlen in den Quetschvorrichtungen wird das Fruchtgewebe vor dem Pressen optimal aufgeschlossen. Dies erlaubt eine hohe Saftausbeute und ein schnelleres Keltern.

Um die Genauigkeit der statistischen Probennahme zu verbessern, darf das Probengut nicht lokal aus dem Traubengut entnommen werden, sondern es muß so entnommen werden, daß es aus unterschiedlichen Bereichen eines Weinbergs oder auch mehrerer Weinberge eines Anlieferers stammende Trauben statistisch hinreichend genau erfaßt. Hierzu sieht die Erfindung in einer äußerst bevorzugten Ausbildung vor, daß der ausgepreßte Saft der Probe vor der Extraktbestimmung durchmischt wird, wobei insbesondere das angelieferte Traubengut kontiniuierlich zur Weiterverarbeitung gefördert wird, daß über die gesamte Zeit der Förderung eine Probe an Beeren abgezweigt, gepreßt und anschließend der Extraktgehalt des ausgepreßten Saftes der Beeren der Probe bestimmt wird. Um dies zu erreichen, ist eine erfindungsgemäße Vorrichtung gekennzeichnet durch eine Fördereinrichtung zum kontinuierlichen Fördern des angelieferten Traubengutes, eine Probennahmeeinrichtung zum Abzweigen einer statistisch repräsentativen Probe von Beeren über die gesamte Zeit der Förderung des Traubengutes, durch eine Probenpreßeinrichtung zum vollständigen Auspressen des Traubengutes, durch eine Meßeinrichtung zur Bestimmung des Extraktgehaltes einer durch Auspressen der Beeren erhaltenen Saftprobe und insbesondere dadurch, daß eine Fördereinrichtung zum Fördern des Traubengutes einen Förderquerschnitt höchstens in der Größenordnung der Abmessung einer Traube aufweist.

Sofern eine Einmalmessung des Mostvorlaufs und des Probenpreßsaftes vorgenommen wird, müssen diese Säfte vor der Messung besonders gründlich gemischt werden.

Durch diese Maßnahmen wird sichergestellt, daß Trauben oder Weinbeeren bei einem nicht homogenen Traubengut mit unterschiedlich verteilten Trauben unterschiedlicher Qualität in einer Weise entnommen werden, daß das als Probe entnommene Traubengut eine repräsentative Probe des Gesamttraubengutes darstellt.

Um eine hinreichende Validität der Probe mit einem ausreichenden Vertrauensniveau zu nehmen, sieht die Erfindung vor, daß als Probe mindestens 1,5 % des Gewichts der angelieferten Charge der Trauben genommen werden und eine Probennahmeeinrichtung dementsprechend ausgebildet ist. Hierdurch wird der durch die Probennahme gegebene statistische Meßfehler in eine Größenordnung gedrückt, die vertretbar ist. Der Meßfehler führt insbesondere nicht zu einer Abweichung des Mostübergewichts gegenüber Wasser, die durch den Extraktgehalt bedingt ist, in Bereichen von mehreren Prozent bzw. zu einer Abweichung von mehreren Grad Öchsle (was oftmals als Bestimmungsmaß für den Extraktgehalt verwendet). Der stichprobenauswahlbedingte Meßfehler liegt vielmehr nur im Rahmen der Genauigkeit der Extraktgehaltangabe (beispielsweise in Öchsle) selbst. So ergibt sich bei einer Probe von 2 % des gesamten Traubengutes ein Meßfehler von 0,26 °Oechsle (entsprechend dem Übergewicht eines Liter Mostes gegenüber Wasser aufgrund des Extraktgehaltes) mit einem Vertrauensniveau von 95 %.

Während es grundsätzlich möglich ist, zur Probennahme einzelne Beeren von ganzen Trauben abzuzweigen, beispielsweise abzureißen oder abzuschneiden oder einen Teil der Trauben zu nehmen, sieht, soweit die Probennahme im Förderweg ganzer Trauben erfolgt, die Erfindung in bevorzugter Ausgestaltung vor, daß die Trauben in einem kontinuierlichen Förderstrom geringen Querschnitts, der insbesondere höchstens der Größenordnung der Abmessungen einer Traube entspricht, zur Weiterverarbeitung gefördert werden.

Durch das erfindungsgemäße Verfahren erfolgt eine statistisch einwandfreie Bestimmung des Zuckergehaltes der angelieferten Trauben, indem über die gesamte Zeit der Zuführung einer Charge von Trauben hin eine repräsentative Probe von Weintrauben entnommen wird, deren Beeren in gleicher Weise (vollständig) ausgepreßt werden, und dann der Zuckergehalt dieser entnommenen Probe bestimmt wird. Hierdurch wird also vermieden, daß überreife Beeren überrepräsentiert sind. Es wird sichergestellt, daß eine statistisch einwandfreie Probe der Beerengesamtheit entnommen wird und damit überreife, normal reife und weniger reife Beeren der Charge statistisch richtig in der Probe repräsentiert sind. Durch das Pressen der Trauben der Probe in gleicher Weise, wie das Pressen des gesamten Ensembles später in der Traubenpresse erfolgt, wird darüber hinaus sichergestellt, daß die Zuckerbestimmung nicht dadurch verfälscht wird, daß nur Saft aus dem Außenbereich der Beeren, also unmittelbar unterhalb der Traubenhaut, entnommen wird. Es wird vielmehr der Saft der gesamten Beeren ausgepreßt und damit ebenfalls eine richtige Zuckerbestimmung erreicht.

Durch die erfindungsgemäße Vorrichtung wird eine solche statistisch einwandfreie Bestimmung des Zuckergehalts unmittelbar beim Anliefern einer Charge von Trauben ermöglicht.

In bevorzugter Ausgestaltung sieht das erfindungsgemäße Verfahren weiter vor, daß einzelne Trauben mittels einer an einer Förderschnecke seitlich angeordneten Zellenradschleuse entnommen werden. Das Pressen der Beeren kann in unterschiedlicher Weise erfolgen. In bevorzugter Ausgestaltung ist vorgesehen, daß die Preßeinrichtung zum Pressen der Trauben der Probe Quetschventile aufweist, so daß die abgezweigten Beeren mittels Quetschventilen gepreßt werden. Statt dessen können auch andere schnell arbeitende und gut zu reinigende Pressen, wie insbesondere hydraulische Pressen, eingesetzt werden. Eine weitere bevorzugte Ausgestaltung der Erfindung sieht vor, daß unterhalb der Preßeinrichtung ein Überlauf mit einem ringförmigen Überlaufblech angeordnet ist, unterhalb dessen sich ein ringförmiger Saftauffangbehälter befindet, an dem seitlich eine Einrichtung zur Bestimmung des Zuckergehaltes angeordnet ist, so daß der ausgepreßte Saft in einem Auffangbehälter aufgefangen und sein Zuckergehalt mittels eines diesem zugeordneten Gerätes zur Bestimmung des Zuckergehaltes (Öchsle-Meßgerät) bestimmt wird, wobei der Zuckergehalt insbesondere refraktometrisch oder auch mittels eines Biegeschwingers durch einmaliges oder kontinuierliches Messen bestimmt wird.

Die erfindungsgemäße Vorrichtung zur Bestimmung des Zuckergehaltes kann bei der Anlieferungstation der Kellerei in unterschiedlicher Weise angeordnet sein. Wenn ein Abbeeren und Quetschen der Trauben nicht erfolgen soll, so sieht eine bevorzugte Ausgestaltung vor, daß die Einrichtung zur Entnahme einer repräsentativen Probe von Beeren unmittelbar einem Einfülltrichter einer Traubenannahmestation zugeordnet ist. Ist eine Abbeermaschine vorhanden, so ist bevorzugterweise vorgesehen, daß die Einrichtung zur Entnahme einer repäsentativen Probe von Beeren in eine Trauben-Abbeermaschine integriert ist. In jedem Falle ist es vorteilhaft, die Einrichtung zur repräsentativen Probenentnahme unmittelbar an einer entsprechenden Förderschnecke, sei es des Einfülltrichters, sei es der Trauben-Abbeermaschine, vorzusehen.

Wenn die Probennahme aus der Maische nach Abbeeren des gesamten Traubengutes und Anquetschen durch das Fördern mittels der Maischepumpe aus einer entsprechenden Förderleitung erfolgt - wobei dann die Probe selbstverständlich die Probe selbst in der beanspruchten Weise ebenfalls mit einem dem Kellerpreßdruck vergleichbaren Preßdruck ausgepreßt werden muß, so kann der sich bei der Anlieferung im Aufnahmebehälter, insbesondere einem Trichter ansammelnde Saft der Maische zugeleitet werden, so daß er mit hinreichender Genauigkeit unmittelbar bei der Probe miterfaßt wird. Wenn, wie dies bevorzugt wird, die Probennahme noch vor dem Abbeeren erfolgt, da dies ja eine gewisse Zeit braucht, so weist die Probe in der Regel keine freien, also von vorneherein vorhandenen Saftanteile im richtigen Verhältnis auf, da diese, wie gesagt, sich am Behälterboden ansammeln und durch einen Ablauf entnommen werden müssen. Die Erfindung sieht daher insbesondere in diesem Fall in bevorzugter Ausgestaltung vor, daß als Probe mindestens 1,5 % des Gewichts der angelieferten Gesamtcharge der Trauben genommen werden, wobei die Vorrichtung sich hierzu auszeichnet durch eine an einem Traubenannahmebehälter angeordnete Saftentnahmeeinrichtung sowie sich an diese anschließende Wiege- und Saftextraktmeßeinrichtungen und durch eine Einrichtung zur Bestimmung des Extraktgehaltes des angelieferten Traubengutes aus den Extraktgehalten der Probe und des durch die Saftentnahmeeinrichtung entnommenen vorhandenen Saftes unter Berücksichtigung des Anteils des vorhandenen Saftes an der angelieferten Charge. An denselben Stellen können aus denselben repräsentativen Proben auch weitere wichtige Mostextraktanteile, z.B. die Gesamtsäure (durch Titration) bestimmt werden. Zunehmend interessieren auch Stoffe, die die Gesundheit von Mensch und Beeren betreffen, z.B. Spritzmittelrückstände und Pilze.

Um schließlich eine Verfälschung der Probenmessung einer Probe durch die vorangehende auszuschließen, ist in weiterer Ausgestaltung vorgesehen, daß neben der Bestimmung des Gesamtgewichtes der angelieferten Gesamtcharge der Trauben auch das Gewicht und der Extraktgehalt des bei der Anlieferung der Trauben schon vorhandenen Saftes gemessen und der Extraktgehalt der Charge aus den Extraktgehalten der Probe und des vorhandenen Saftes unter Berücksichtigung des Anteils des vorhandenen Saftes an der angelieferten Charge bestimmt werden, wobei hierzu eine an einem Traubenannahmebehälter angeordnete Saftentnahmeeinrichtung sowie sich an diese anschließende Wiege-/Volumen- und Saftextraktmeßeinrichtungen und eine Einrichtung zur Bestimmung des Extraktgehaltes des angelieferten Traubengutes aus den Extraktgehalten der Probe und des durch die Saftentnahmeeinrichtung entnommenen vorhandenen Saftes unter Berücksichtigung des Anteils des vorhandenen Saftes an der angelieferten Charge vorgesehen sind.

Die Reinigung kann dabei prinzipiell mit Wasser oder Dampf erfolgen, wobei zum Trocknen Druckluft durch das System geblasen werden kann. Die Reinigung wird erheblich erleichtert, wenn wesentliche Teile des Probennahmesystems aus PTFE bestehen bzw. mit diesem Material beschichtet sind, da dieses Material flüssigkeitsabweisend ist.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnung im einzelnen erläutert ist. Dabei zeigt:
- Fig. 1: den Ablauf der Verarbeitung von Weintrauben;
- Fig. 2: eine Seitenansicht (aufgeschnitten) einer Trauben-Abbeermaschine mit einer Einrichtung zu einer repräsentativen Probenentnahme von Wein-Beeren gemäß der Erfindung;
- Fig. 3: einen Schnitt entsprechend III-III durch die Vorrichtung der Fig. 2;
- Fig. 4: wesentliche Teile einer anderen bevorzugten Ausgestaltung der erfindungsgemäßen Vorrichtung in Seitenansicht; und
- Fig. 5: ebenfalls wesentliche Teile einer weiteren bevorzugten Ausgestaltung der erfindungsgemäßen Vorrichtung in Seitenansicht.

Geerntete Weintrauben werden zunächst mittels eines Fahrzeugs 1 zur Traubenannahme angeliefert und von dem Fahrzeug 1 in einen Einfülltrichter 2 eingefüllt. Dieser weist eine Förderschnecke 2a auf, mit der die Trauben zum Einlaßtrichter 3a einer Trauben-Abbeermaschine 3 gefördert werden. In dieser Trauben-Abbeermaschine 3 erfolgt ein Abbeeren, d.h. Trennen der Wein-Beeren von den Kämmen, und gegebenenfalls ein leichtes Quetschen der Beeren. Die Kämme werden durch einen Kammentsorgungs-Förderer 4 entfernt. Die Wein-Beeren fallen in einen Wiegebehälter 5, der auf einem Wiegesystem 6 gelagert ist, das mit einer Auswerteeinheit 8 verbunden ist. An der Unterseite des Wiegebehälters 5 ist ein Verschluß 7 vorgesehen, der durch einen Flachschieber, ein Quetschventil oder dgl. ausgebildet sein kann.

Die gewogenen Trauben fallen durch Öffnen des Verschließorgans 7 in einen Trauben- und Maischetrichter 9 mit einer Exzenterschneckenpumpe 9a, die über Rohrleitungen 11 die Beeren zu einer Traubenpresse 16 fördert, an die sich Saft- und Weinpumpen 17 einerseits, andererseits eine Tresterentsorgung 18 in Form einer Förderschnecke oder eines Förderbandes anschließt.

Hinter der Traubenpresse 16 wird der Zuckergehalt des zur Weiterverarbeitung als Wein vorgesehenen Traubensaftes bestimmt, der u.a. die Art der Weiterverarbeitung des Saftes bestimmt, aber auch die Klassifizierung des herzustellenden Weines.

Da einerseits die Verarbeitung des angelieferten Traubengutes von der Anlieferung bis zur Traubenpresse 16 eine gewisse Zeit beansprucht und gegebenenfalls eine Zwischenlagerung angelieferten Gutes in Abtropftanks oder Gärtanks und im übrigen bis zur Traubenpresse insbesondere bei Winzergenossenschaften eine Zusammenführung von Chargen von Trauben unterschiedlicher Anlieferer bzw. Weinbauern erfolgt, deren Vergütung aber andererseits nach Gewicht und der Qualität des Traubengutes, d.h. dem Zuckergehalt (selten zusätzlich nach dem Gesamtsäureanteil), berechnet wird und diese daher den Zuckergehalt ihres Traubengutes sofort nach Anlieferung wissen möchten, wurde bisher aus dem sich im Wiegebehälter oder unter der Abbeermaschine befindlichen Saft mittels eines Schlauches eine Probe herausgesogen und refraktometrisch der Zuckergehalt dieser Probe bestimmt. Der Saft kann durch das Aufplatzen sehr reifer Trauben, das Abbeeren oder auch das Vorquetschen der Trauben entstanden sein.

Da sich herausgestellt hat, daß diese Zuckergehalt-Bestimmung unmittelbar nach der Anlieferung des Traubengutes, d.h. die Bestimmung der sogenannten Annahmestations-Öchsle, zu falschen Werten führt, insbesondere gegenüber der Bestimmung des nach dem Pressen der Trauben hinter der Traubenpresse 16 bestimmten sogenannten maßgebenden Keller-Öchsles, wobei die Werte um 5-6 Öchsle abweichen können, schlägt die Erfindung unmittelbar nach der Traubenanlieferung eine repräsentative Probenentnahme von Beeren vor.

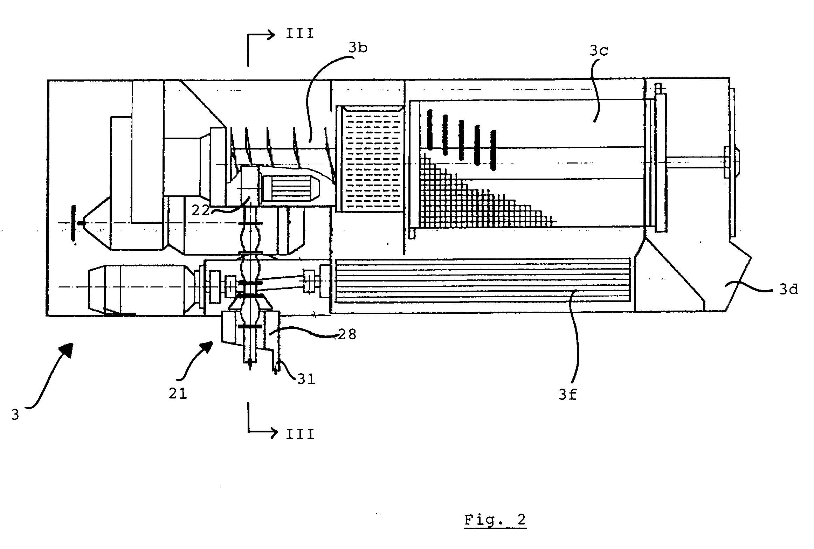

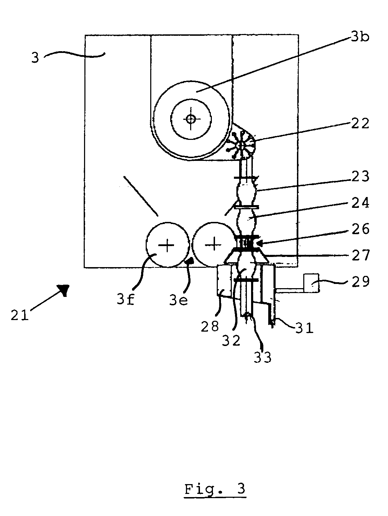

Hierzu beinhaltet die Erfindung eine Vorrichtung 21 zum Bestimmen des Zuckergehalts (und erforderlichenfalls der Gesamtsäure) einer Charge von Trauben bei der Anlieferung mit einer Einrichtung 22 zur repräsentativen Probenentnahme von Beeren der Trauben (Fig. 2 und 3).

Bei dem dargestellten Ausführungsbeispiel ist die Vorrichtung zum Bestimmen des Zuckergehalts in die Trauben-Abbeermaschine 3 integriert, wobei die Probennahmeeinrichtung 22 an einer Förderschnecke 3b unterhalb des Einlaßtrichters 3a der Trauben-Abbeermaschine 3 angeordnet ist.

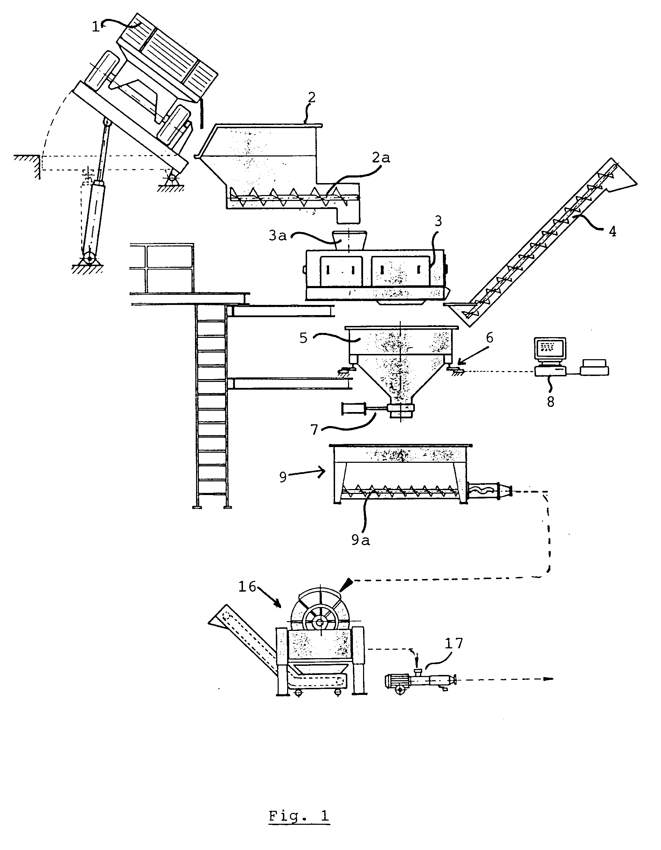

Wenn ein Abbeeren der Trauben nicht erfolgen soll, also eine Trauben-Abbeermaschine nicht vorhanden ist, weil die Trauben bzw. Beeren vor dem endgültigen Pressen mechanisch so wenig wie möglich bearbeitet werden sollen, so kann die erfindungsgemäße Vorrichtung zum Bestimmen des Zuckergehaltes in gleicher Weise auch der Förderschnecke 2a des Einfülltrichters zugeordnet sein (Fig. 1).

An die Förderschnecke 3b der Trauben-Abbeermaschine 3 schließt sich eine Abbeer-Einrichtung 3c an, in der die Beeren von den Kämmen der Trauben abgestreift werden. Die Kämme werden über einen Auslaß 3d aus der Abbeer-Einrichtung 3c zur Kammentsorgung 4 herausgefördert, während die Beeren zum Spalt 3e (Fig. 3) von Quetschwalzen 3f herunter- und gegebenenfalls durch diesen hindurchfallen, wenn der Abstand der Quetschwalzen hinreichend groß eingestellt ist. Der Abstand der Quetschwalzen 3f kann in gewünschter Weise eingestellt werden, so daß die Beeren entweder überhaupt nicht oder leicht gequetscht werden.

Die erfindungsgemäße Vorrichtung 21 zur Bestimmung des Zuckergehalts von Trauben weist eine Einrichtung 22 zur repräsentativen Probenentnahme auf, die im dargestellten Ausführungsbeispiel durch eine Zellenradschleuse gebildet ist, die unmittelbar am Umfang der Förderschnecke 3b angeordnet ist. Unterhalb der Zellenradschleuse 22a befinden sich Quetschventile 23 und 24. An letzteres schließt sich ein Austritt 26 für aus den Beeren ausgepreßten Saft an, der über ein ringförmiges Überlaufblech 27 in einen unter ihm angeordneten, ebenfalls ringförmigen Saftauffangbehälter 28 fließt, an dem seitlich ein Gerät 29 zur Bestimmung des Zuckergehaltes, also ein Öchsle-Meßgerät, vorzugsweise ein refraktometrisches, angeordnet ist. An der Unterseite des Saftauffangbehälters 28 befindet sich ein verschließbarer Auslaß. Unterhalb der Austrittsstelle 26 befindet sich ein weiteres Quetschventil 32, welches zu einem Tresterablauf 33 führt.

Mittels der Förderschnecke 3b werden Trauben aus Beeren und Kämmen gefördert. Die Zellenradschleuse 22 ist derart dimensioniert, daß sie über die gesamte Zuförderzeit einer Charge hin ganze Trauben aus der Förderschnecke 3b abzweigt - wobei sie auch einzelne Beeren und schon ausgepreßten Saft mit aufnehmen kann - und diese zu den Quetschventilen 23, 24 fördert, mittels derer die Trauben ausgepreßt werden. Der dabei erzeugte Druck kann in der Größenordnung von 2-4 bar liegen, entspricht also dem Preßdruck, mit dem die Beeren später in der Traubenpresse 16 (Fig. 1) ausgepreßt werden. Der ausgedrückte Saft fließt in den Saftauffangbehälter 28 und tritt, nachdem eine hinreichende Menge von Saft sich in diesem angesammelt hat, in das seitlich angeordnete Gerät 9 zur Bestimmung des Zuckergehaltes, womit der Zuckergehalt der entnommenen repräsentativen Probe von Beeren und damit der entsprechenden zugelieferten Charge mit hoher Genauigkeit bestimmt werden kann.

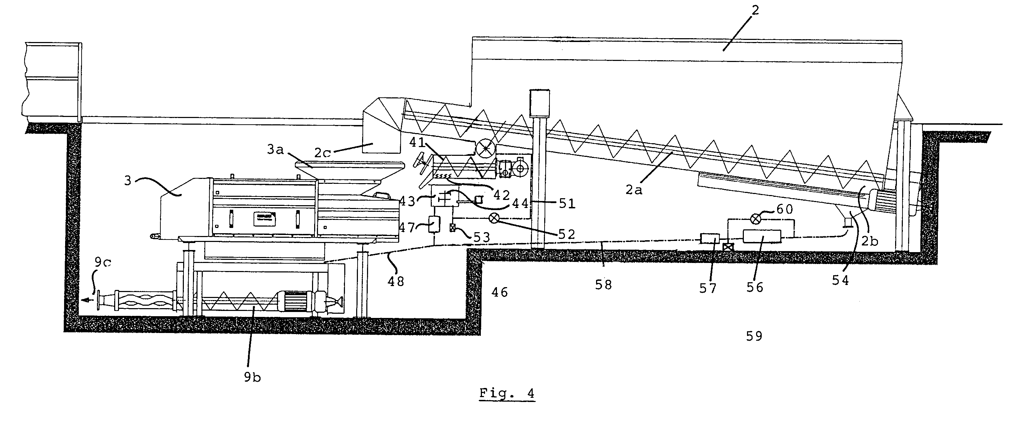

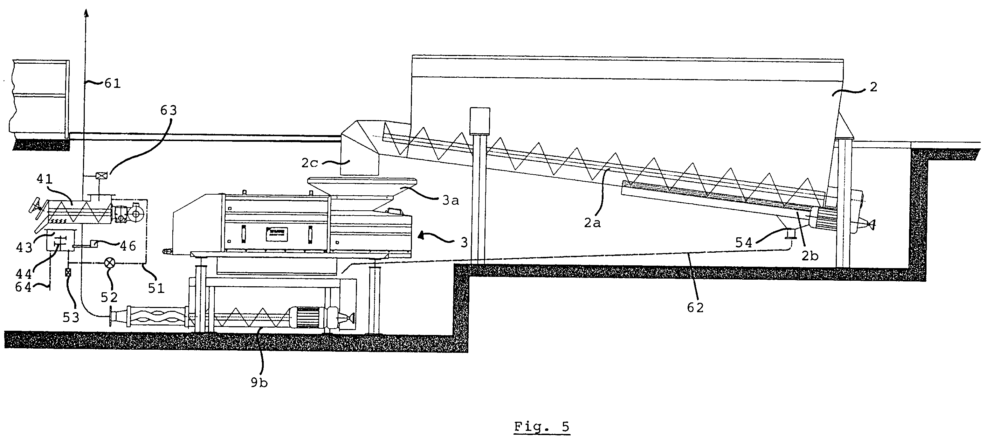

Gleiche Teile sind auch in den Fig. 4 und 5 mit den gleichen Bezugszeichen wie in den Fig. 1 bis 3 bezeichnet. Ein Aufnahmebehälter oder Einfülltrichter 2 weist in seinem Bodenbereich eine Förderschnecke 2a auf, mit der auch hier die Trauben zu einem Einlaßtrichter 3a einer Traubenabbeermaschine 3 gefördert werden. Die Kämme werden in bekannter, nicht näher dargestellter Weise entfernt. Ein Wiegesystem ist hier in der Traubenabbeermaschine 3 integriert und ebenfalls nicht näher dargestellt. Aus der Traubenabbeermaschine 3 fallen die Trauben in eine Maischepumpe 9b und werden von dort über deren Auslaß 9c in gleicher Weise wie aus der Fig. 1 ersichtlich zur Weiterverarbeitung gefördert (dort über die Leitung 11).

Die Förderschnecke 2a ist geneigt angeordnet und fördert die Trauben aus einem tieferen Bereich 2b des Einfülltrichters 2 schräg nach oben zum Auslaß 2c der Förderschnecke 2a über dem Trichter 3a. Vor dem Auslaß 2c ist wiederum eine Einrichtung zur repräsentativen Probennahme 22 in Form einer Zellenradschleuse angeordnet, die einen bestimmten Prozentsatz der durch die Schnecke 2a geförderten Trauben aus deren Förderweg entnimmt, wobei vorzugsweise vollständige Trauben entnommen werden, und sie in eine Probenpresse 41 gibt. Die Probenpresse 41 ist vorzugsweise unterhalb oder schräg unterhalb der Förderschnecke 2a angeordnet. Die Probenpresse 41 preßt die Trauben der Probe so, daß sich derselbe Ausbeutegrad ergibt wie beim späteren Pressen der Gesamtheit der Trauben in der Traubenpresse 16 (Fig. 1). Der Saft tropft durch Öffnungen 42 der Probenpresse 41 in einen Probenbehälter 43, in dem ein Rührwerk 44 angeordnet ist, vorzugsweise in Form eines Magnetrühres, um den im Behälter 43 aufgenommenen Saft gut zu durchmischen, so daß tatsächlich der durchschnittliche Extraktgehalt bestimmt werden kann. Hierzu ist seitlich des Behälters 43 eine Extrakt-Meßeinrichtung 46 angeordnet, die beispielsweise als Refraktometer oder als automatische Extraktbestimmungseinrichtung ausgebildet sein kann. Der aus der Probe ausgepreßte Traubensaft fließt dann über eine Durchflußmeßeinrichtung 47 und eine Leitung 48 zur Maischepumpe 9b.

Weiterhin ist im Bereich der vorbeschriebenen Einrichtungen 41 bis 43 zur Probennahme und -bestimmung ein Spülkreislauf 51 mit einer Umwälzpumpe 52 und einem Auslaß 53 vorgesehen, mit dem die Probennahme- und Meßeinrichtungen gespült und so für die Aufnahme und Bestimmung einer weiteren Probe einer unterschiedlichen Charge vorbereitet werden können.

Im unteren Bereich 2b des Aufnahmetrichters 2 findet sich ein Saftauslauf 54, dem sich eine weitere Extrakt-Meßeinrichtung 56 anschließt, die hier als Extrakt-Durchflußmeßeinrichtung ausgebildet ist. An diese schließt sich wiederum ein Durchflußmesser 57 an. Der bei 54 entnommene Saft wird dann über eine weitere Leitung 58 der Leitung 48 und über diese der Maischepumpe 9b zugeführt. Auch der Extrakt-Meßeinrichtung 56 ist ein Spülkreislauf 59 mit einer Pumpe 60 zugeordnet.

Eine Charge von Trauben wird angeliefert und in den Trichter 2a geschüttet. Die Trauben werden durch die Förderschnecke 2a zum Auslaß 2c und über diesen in die Traubenabbeermaschine 3 gefördert. Über die gesamte Förderzeit, mit der die Trauben der Charge aus dem Trichter 2 mittels der Förderschnecke 2a herausgefördert werden, nimmt die Probennahmeeinrichtung 22 in Form der Zellenradschleuse laufend Proben, indem sie in genau vorbestimmter Weise Trauben aus der Förderschnecke 2a abzweigt, und zwar ist sie derart eingestellt, daß sie mindestens 1,5, vorzugsweise aber 2 % der Trauben, also jede fünfzigste hindurchgeförderte Traube entnimmt. Dadurch, daß die Trauben durch die Förderschnecke 2a nur mit einem Förderstrom mit geringem Querschnitt gefördert werden, der in der Größenordnung der Traubenabmessungen liegt, und sich damit ein langer Förderstrom ergibt, wird erreicht, daß durch die über die gesamte Förderzeit hin erfolgende Probennahme tatsächlich eine statistisch einwandfreie Probennahme erfolgt und nicht etwa für die Probe nur Trauben genommen werden, die beim Eingeben der Charge in den Trichter 2 sich in einem eng definierten Bereich desselben befinden und demgemäß mit hoher Wahrscheinlichkeit aus einem ganz bestimmten Bereich des abgeernteten Weinbergs stammen. Durch die Förderung und die Entnahme aus dem Förderweg erfolgt vielmehr eine Verteilung der Trauben und damit eine Entnahme aus allen Bereichen, wodurch eine statistisch hinreichende Probennahme gewährleistet ist.

Die Trauben werden sodann mittels der Probenpresse 41 gepreßt und, wie gesagt, in der gleichen Weise, wie dies später für die Gesamtcharge in der Traubenpresse erfolgt. Auch hierdurch wird eine Vergleichbarkeit des der nachfolgenden Probenextraktmessung zugrunde liegenden Mostes oder Saftes mit dem später aus der Gesamtcharge ausgepreßten Saft und damit eine statistischen Anforderungen genügende Probenmessung gewährleistet. Dies wird noch dadurch unterstützt, daß der aufgefangene Saft der Probe gut durchmischt wird und erst anschließend die Bestimmung des Extraktgehaltes durch die Meßeinrichtung 46 erfolgt.

Damit das Meßergebnis einer nachfolgend genommenen Probe nicht durch Restbestände der zuvor gemessenen Probe verfälscht wird, erfolgt anschließend eine Spülung der gesamten mit der Probe vor der Bestimmung des Extraktgehaltes in Berührung kommenden Teile über den Spülkreislauf 51, indem beispielsweise Wasser oder Dampf durch die Pumpe 52 hindurchgedrückt wird. Weiterhin kann ein Trocknen der Teile 41, 42, 43 erfolgen.

Beim Anliefern von Trauben werden nicht nur vollständige Trauben angeliefert, sondern aus verschiedensten Gründen schon geplatzte oder angequetschte Trauben, aus denen dann auch Saft austritt. Dieser kann über die Zellenradschleuse nicht in statistisch einwandfreier Form mitentnommen werden. Er sammelt sich vielmehr im unteren Bereich des Trichters 2 an. Darüber hinaus wird dieser vorab ausgequetschte Saft einen anderen Extrakt- oder Zuckergehalt aufweisen als die gesamte Traubenmenge, und zwar deswegen, weil der Extraktgehalt über den Querschnitt einer Traube und über den Querschnitt einer einzelnen Beere hin nicht gleich ist und der vorab ausgelaufene Saft weitgehend aus äußeren Bereichen der Traube und dabei außenliegenden Bereichen äußerer Beeren stammt. Um die Genauigkeit der Vorabbestimmung des Extraktgehaltes zu erhöhen, ist daher vorgesehen, daß der Extraktgehalt dieses Saftes ebenfalls unabhängig bestimmt wird, was in der Meßeinrichtung 56 erfolgt. Aus den beiden über die Einrichtungen 46 und 56 gemessenen Werten wird dann unter Berücksichtigung der Menge des Saftes (gemessen über die Einrichtung 57), der Menge des Probensaftes (gemessen über die Einrichtung 47) - wenn der Anteil der entnommenen Trauben aus der Gesamtmenge der Trauben genau definiert ist - und/oder aus der Menge der Gesamttrauben - gewogen durch die der Traubenabbeermaschine zugeordnete (hier nicht weiter, aber in Fig. 1 dargestellte) Wiegeeinrichtung - der Extraktgehalt der Trauben bestimmt.

Bei der Ausgestaltung der Fig. 5 wird die Probe aus einer von der Maischepumpe 9b zur Traubenpresse (Fig. 1) führenden Leitung 61 abgezweigt. Es ist daher zunächst möglich, den im unteren Bereich 2b des Aufnahmetrichters 2 sich sammelnden Most über den Ablaß 54 und eine Leitung 62 direkt der Maischepumpe 9b zuzuführen.

Die Einrichtung 63 zur Probennahme der Fig. 5 weist ein Dosierventil auf, mittels dessen dosiert Maische aus der Leitung 61 abgezweigt wird, und zwar wiederum mindestens 1,5, vorzugsweise 2 % des gesamten Maischedurchflusses. Die abgezweigte Maische wird einer Probenpresse 41 zugeführt, die den gesamten Saft aus der Maische, auch den aus den angequetschten Trauben entsprechend der Bedingungen, die in der Traubenpresse herrschen, aus- und in einen Probebehälter 43 eindrückt. In dem Probenbehälter 43 findet sich wieder ein Rührwerk 44 und eine Extraktmeßeinrichtung 46, mit der der Extraktgehalt der Probe bestimmt wird. Die Probe, deren Extraktgehalt bestimmt ist, kann über einen Saftablauf 64 fortgeführt werden. In gleicher Weise wie bei der Ausgestaltung der Fig. 4 ist noch ein Spülkreislauf 51 mit einer Pumpe 52 und einem Auslaß 53 vorhanden.

Auch bei der Ausgestaltung der Fig. 5 werden die in den Trichter 2 eingehenden Trauben durch die Förderschnecke 2a über den Auslaß 2c zur Traubenabbeermaschine 3 und von dort in die Maischepumpe 9b gefördert. In dieser wird, wie gesagt, der sich im unteren Bereich 2b des Trichters 2 ansammelnde Saft über die Leitung 62 zugeführt. Die Maischepumpe 9b fördert die Maische durch die Leitung 61 zur Traubenpresse (die in Fig. 1 dargestellt ist). Über die Probennahmeeinrichtung in Form des Dosierventils wird eine Probe genommen und in der unter Bezugnahme auf die Fig. 4 beschriebenen Weise ihr Extraktgehalt bestimmt. Nach Auslaß des Probensaftes erfolgt ein Spülen in der ebenfalls schon beschriebenen Weise.

## Patentansprüche

1. Verfahren zum Bestimmen des Extraktgehaltes von Traubengut bei der Anlieferung zur Verarbeitung zu Traubenmost, wobei eine Probe des angelieferten Traubengutes genommen wird, diese gepreßt wird und anhand des ausgepreßten Saftes der Probe der Extraktgehalt bestimmt wird, dadurch gekennzeichnet, daß die Probe mit einem wesentlich höheren Proben-Preßdruck gegenüber dem Preßdruck ausgepreßt wird, mit dem die gesamte angelieferte Charge des Traubengutes, deren Extraktgehalt vorab bestimmt werden soll, zum Erhalt des Traubenmostes gepreßt wird.

2. Verfahren nach Anspruch 1, dadurch gekennzeichnet, daß ein dünner Preßkuchen erzeugt wird.

3. Verfahren nach Anspruch 1 oder 2, dadurch gekennzeichnet, daß die Probe kontinuierlich gepreßt wird.

4. Verfahren nach Anspruch 1 oder 2, dadurch gekennzeichnet, daß die Probe diskontinuierlich gepreßt wird.

5. Verfahren nach einem der vorangehenden Ansprüche, dadurch gekennzeichnet, daß der aus der Probe ausgepreßte Saft nach Bestimmung seines Extraktgehaltes dem Traubengut, zu dessen Vorabbestimmung des Extraktgehaltes er diente, wieder zugeführt wird.

6. Verfahren nach einem der vorangehenden Ansprüche, dadurch gekennzeichnet, daß die Beeren der Probe vor dem Auspressen von ihren Kämmen abgebeert werden.

7. Verfahren nach einem der vorangehenden Ansprüche, dadurch gekennzeichnet, daß der ausgepreßte Saft der Probe vor der Extraktbestimmung durchmischt wird.

8. Verfahren insbesondere nach einem der vorangehenden Ansprüche, dadurch gekennzeichnet, daß das angelieferte Traubengut kontiniuierlich zur Weiterverarbeitung gefördert wird, daß über die gesamte Zeit der Förderung eine Probe an Beeren abgezweigt, gepreßt und anschließend der Extraktgehalt des ausgepreßten Saftes der Beeren der Probe bestimmt wird.

9. Verfahren nach einem der vorangehenden Ansprüche, dadurch gekennzeichnet, daß die Trauben in einem kontinuierlichen Förderstrom geringen Querschnitts, der insbesondere höchstens der Größenordnung der Abmessungen einer Traube entspricht, zur Weiterverarbeitung gefördert werden.

10. Verfahren nach einem der vorangehenden Ansprüche, dadurch gekennzeichnet, daß über die Zeit der Zuführung einzelne Trauben abgezweigt werden.

11. Verfahren nach einem der vorangehenden Ansprüche, dadurch gekennzeichnet, daß die Trauben mittels einer an einer Förderschnecke für die Trauben angreifenden Zellenradschleuse abgezweigt werden.

12. Verfahren nach einem der vorangehenden Ansprüche, dadurch gekennzeichnet, daß der ausgepreßte Saft in einem Auffangbehälter aufgefangen und sein Extraktgehalt mittels eines diesem zugeordneten Gerätes zur Bestimmung des Extraktgehaltes bestimmt wird.

13. Verfahren nach Anspruch 12, dadurch gekennzeichnet, daß der Extraktgehalt refraktometrisch bestimmt wird.

14. Verfahren nach einem der vorangehenden Ansprüche, dadurch gekennzeichnet, daß als Probe mindestens 1,5 % vom Gewicht der angelieferten Gesamtcharge der Trauben genommen werden.

15. Verfahren insbesondere nach einem der vorangehenden Ansprüche, dadurch gekennzeichnet, daß neben der Bestimmung des Gesamtgewichtes der angelieferten Gesamtcharge der Trauben auch das Gewicht und der Extraktgehalt des bei der Anlieferung der Trauben schon vorhandenen Saftes gemessen und der Extraktgehalt der Charge aus den Extraktgehalten der Probe und des vorhandenen Saftes unter Berücksichtigung des Anteils des vorhandenen Saftes an der angelieferten Charge bestimmt werden.

16. Verfahren nach einem der vorangehenden Ansprüche, dadurch gekennzeichnet, daß die von der entnommenen Probe durchlaufenen Einrichtungen nach jeder Probenmessung gereinigt werden.

17. Vorrichtung zum Bestimmen des Extraktgehaltes von Traubengut bei der Anlieferung zur Verarbeitung zu Traubenmost, mit einer Probennahmeeinrichtung (22, 63) zum Nehmen einer Probe des angelieferten Traubengutes, mit einer Probenpreßeinrichtung zum Pressen der Probe und mit einer Meßeinrichtung (29, 46) zur Bestimmung des Extraktgehaltes der Probe, gekennzeichnet durch eine mit einem gegenüber dem Preßdruck, mit dem die angelieferte Charge des Traubengutes zum Erhalt des Traubenmostes gepreßt wird, wesentlich höheren Proben-Preßdruck arbeitende Probenpreßeinrichtung (24, 41).

18. Vorrichtung nach Anspruch 17, gekennzeichnet durch eine kontinuierlich arbeitende Probenpreßeinrichtung (41).

19. Vorrichtung nach Anspruch 17, gekennzeichnet durch eine diskontinuierlich arbeitende Probenpreßeinrichtung (42).

20. Vorrichtung nach einem der Ansprüche 17 bis 19, gekennzeichnet durch eine von der Probenpreßeinrichtung zum Förderweg der Trauben führende Leitung.

21. Vorrichtung nach einem der Ansprüche 17 bis 20, gekennzeichnet durch eine der Probenpreßeinrichtung vorgeschaltete Mini-Abbeermaschine.

22. Vorrichtung nach einem der Ansprüche 17 bis 21, dadurch gekennzeichnet, daß vor der Meßeinrichtung ein Mischer (44) zum Durchmischen des ausgepreßten Saftes der Probe angeordnet ist.

23. Vorrichtung nach einem der Ansprüche 17 bis 22, dadurch gekennzeichnet, daß die Probennahmeeinrichtung (22, 63) derart ausgebildet ist, daß sie mindestens 1,5 % der Beeren der angelieferten Gesamtcharge des Traubengutes als Probe entnimmt.

24. Vorrichtung insbesondere nach einem der Ansprüche 17 bis 23, gekennzeichnet durch eine Fördereinrichtung (2a, 61) zum kontinuierlichen Fördern des angelieferten Traubengutes, eine Probennahmeeinrichtung (22, 63) zum Abzweigen einer statistisch repräsentativen Probe von Beeren über die gesamte Zeit der Förderung des Traubengutes, durch eine Probenpreßeinrichtung (24, 41) zum vollständigen Auspressen des Traubengutes und durch eine Meßeinrichtung (29, 46) zur Bestimmung des Extraktgehaltes einer durch Auspressen der Beeren erhaltenen Saftprobe.

25. Vorrichtung nach einem der Ansprüche 17 bis 24, dadurch gekennzeichnet, daß eine Fördereinrichtung (2a, 61) zum Fördern des Traubengutes einen Förderquerschnitt höchstens in der Größenordnung der Abmessung einer Traube aufweist.

26. Vorrichtung nach einem der Ansprüche 24 oder 25, dadurch gekennzeichnet, daß die Fördereinrichtung eine Förderschnecke (2a) ist.

27. Vorrichtung nach einem der Ansprüche 17 bis 26, dadurch gekennzeichnet, daß die Probennahmeeinrichtung unmittelbar einem Einfülltrichter (2) einer Traubenannahmestation zugeordnet ist.

28. Vorrichtung nach einem der Ansprüche 17 bis 27, dadurch gekennzeichnet, daß die Probennahmeeinrichtung in eine Traubenabbeermaschine (3) integriert ist.

29. Vorrichtung nach einem der Ansprüche 17 bis 26, dadurch gekennzeichnet, daß die Probennahmeeinrichtung (63) einer Maischepumpe (9b) nachgeordnet ist.

30. Vorrichtung nach einem der Ansprüche 17 bis 29, dadurch gekennzeichnet, daß die Probennahmeeinrichtung (22) eine Zellenradschleuse ist.

31. Vorrichtung nach einem der Ansprüche 17 bis 30, dadurch gekennzeichnet, daß unterhalb der Probenpreßeinrichtung (23, 24) ein Überlauf (26) mit einem ringförmigen Überlaufblech (27) angeordnet ist, unterhalb dessen sich ein ringförmiger Saftauffangbehälter (28) befindet, an dem seitlich die Meßeinrichtung (29) zur Bestimmung des Extraktgehaltes angeordnet ist.

32. Vorrichtung insbesondere nach einem der Ansprüche 17 bis 31, gekennzeichnet durch eine an einem Traubenannahmebehälter (2) angeordnete Saftentnahmeeinrichtung (54) sowie sich an diese anschließende Wiege-/Volumenmeß- und Saftextraktmeßeinrichtungen und durch eine Einrichtung zur Bestimmung des Extraktgehaltes des angelieferten Traubengutes aus den Extraktgehalten der Probe und des durch die Saftentnahmeeinrichtung (54) entnommenen vorhandenen Saftes unter Berücksichtigung des Anteils des vorhandenen Saftes an der angelieferten Charge.

33. Vorrichtung nach einem der Ansprüche 17 bis 32, gekennzeichnet durch eine Reinigungseinrichtung zum Reinigen der Probenpreß- und Meßeinrichtungen (41, 42, 43).
